# EUROPÄISCHE PATENTSCHRIFT

(11) **EP 3 454 940 B1**
(45) Veröffentlichungstag und Bekanntmachung des Hinweises auf die Patenterteilung: **21.07.2021**
(21) Anmeldenummer: 17722747.7
(22) Anmeldetag: 09.05.2017
(51) Int. Cl.: A61N 2/02

(54) **VORRICHTUNG ZUR KERNSPINRESONANZTHERARPIE MIT VERSCHIEBBAREN SEITENTEILEN**
DEVICE FOR NUCLEAR SPIN RESONANCE THERAPY WITH DISPLACEABLE SIDE PARTS
DISPOSITIF DE THÉRAPIE PAR RÉSONANCE MAGNÉTIQUE NUCLÉAIRE COMPORTANT DES PARTIES LATÉRALES MOBILES

(30) Priorität: 10.05.2016 DE 102016108600
(43) Veröffentlichungstag der Anmeldung: 20.03.2019
(73) Patentinhaber: Muntermann, Axel, 35580 Wetzlar-Nauborn (DE)
(72) Erfinder: Muntermann, Axel, 35580 Wetzlar-Nauborn (DE)
(74) Vertreter: Blumbach · Zinngrebe Patentanwälte PartG mbB
(86) Internationale Anmeldenummer: PCT/EP2017/061035
(87) Internationale Veröffentlichungsnummer: WO 2017/194528

(56) Entgegenhaltungen:
- EP-A1- 1 741 467
- WO-A1-2005/075019
- WO-A1-2011/076395
- WO-A2-2009/156117
- CN-A- 104 721 959
- GB-A- 429 044

## Beschreibung

### Gebiet der Erfindung

Die Erfindung betrifft eine Vorrichtung zur Kernspinresonanztherapie, welche verschiebbare Seitenteile aufweist, in denen jeweils eine Spule angeordnet ist.

### Hintergrund der Erfindung

Vorrichtungen zur Kernspinresonanztherapie sind bekannt. Es handelt sich dabei um Geräte, mittels derer innerhalb eines Behandlungsvolumens Kernspinresonanzen im Gewebe des Benutzers erzielbar sind.

Derartige Geräte werden sowohl zu medizinischen Heilungszwecken, beispielsweise bei Arthrose, als auch zu kosmetischen Zwecken, beispielsweise zur Hautstraffung oder zur Behandlung von Cellulite, eingesetzt.

Eine Vorrichtung zur Erzeugung von Kernspinresonanzen im Gewebe des Benutzers ist beispielsweise aus der WO 2011/076395 A1 (Erfinder Axel Muntermann) bekannt.

Die in diesem Dokument dargestellte Vorrichtung besteht aus einer Liege oder einem Stuhl. Um im Behandlungsvolumen Kernspinresonanzen zu erzeugen, weist die Vorrichtung zwei sich vertikal neben dem Stuhl oder der Liege erstreckende Seitenteile auf, in denen jeweils eine Spule angeordnet ist.

Die in den Seitenteilen angeordneten Spulen stehen in Helmholtz-Konfiguration und es wird mittels dieser Spulen ein geradlinig zwischen den Seitenteilen verlaufendes magnetisches Feld erzeugt, dessen Feldstärke innerhalb des Behandlungsvolumens eine gute Homogenität aufweist.

Über eine unter der Liegefläche angeordnete weitere Spule wird ein senkrecht zu vorstehend genanntem Feld stehendes magnetisches Wechselfeld eingestrahlt. Bei Abstimmung der Frequenz des Wechselfeldes auf die Feldstärke des von den Spulen in den Seitenteilen erzeugten Feldes kann die Resonanzbedingung für Kernspinresonanzen erzielt werden.

Vorzugsweise wird das von den Spulen in den Seitenteilen erzeugte magnetische Feld derart moduliert, dass es aus einem Sockelbetrag und einem periodisch geänderten hinzugefügten Betrag zusammengesetzt ist ("gesweept"). Das magnetische Wechselfeld wird zumindest während der fallenden Flanken des gesweepten Feldes eingeschaltet und bei jedem Durchlauf wird temporär die Resonanzbedingung erreicht.

Um die Vorrichtung auf den Benutzer anpassen zu können sowie um unterschiedliche Körperregionen behandeln zu können, umfasst gemäß einer in vorstehendem Dokument dargestellten Ausführungsform die Vorrichtung Spulen, welche in verschiebbaren Seitenteilen angeordnet sind.

Nachteilig ist hierbei, dass die Seitenteile derart verschoben werden können, dass sich diese nicht genau gegenüberstehen. Dies kann Bedienungsfehler nach sich ziehen, die dazu führen, dass sich wegen nicht genau gegenüberliegender Spulen das Behandlungsvolumen reduziert oder dass sogar gar keine Kernspinresonanzen erzielt werden.

Gleichzeitig besteht bei der vorstehend beschriebenen Vorrichtung die Möglichkeit, dass die Vorrichtung einen Defekt hat, beispielsweise, dass eine der Spulen eine Unterbrechung oder einen Kurzschluss zwischen den einzelnen Wicklungen der Spule aufweist, und dass dies vom Verwender der Vorrichtung nicht bemerkt wird.

Die Dokumente WO 2005/075019 A1 und WO 2009/156117 A2 zeigen ebenfalls Therapiegeräte, welche Kernspinresonanzen im zu behandelnden Gewebe erzeugen. Das Dokument EP 1 741 467 A1 zeigt eine Therapieliege, die elektromagnetische Felder erzeugt.

### Aufgabe der Erfindung

Der Erfindung liegt demgegenüber die Aufgabe zugrunde, die Benutzung und Bedienung einer Vorrichtung zur Behandlung mit Kernspinresonanzen einfacher und sicherer zu machen.

### Zusammenfassung der Erfindung

Die Aufgabe der Erfindung wird bereits durch eine Vorrichtung zur Kernspinresonanztherapie nach Anspruch 1 gelöst.

Bevorzugte Ausführungsformen und Weiterbildungen der Erfindung sind dem Gegenstand der abhängigen Ansprüche, der Beschreibung sowie den Zeichnungen zu entnehmen.

Die Erfindung betrifft eine Vorrichtung zur Kernspinresonanztherapie, also eine Vorrichtung, mittels welcher sich, wie eingangs beschrieben, Kernspinresonanzen im zu behandelnden Gewebe eines Benutzers erzeugen lassen.

Die Vorrichtung umfasst eine Liege mit einer vorzugsweise horizontal angeordneten Liegefläche für den Benutzer.

Angrenzend an die Liegefläche weist die Vorrichtung zwei sich gegenüberliegende Seitenteile auf, welche jeweils eine Spule umfassen.

Die Spulen in den Seitenteilen sind insbesondere in Helmholtz-Konfiguration angeordnet und dienen der Erzeugung eines geradlinigen Feldes, welches sich quer über die Liegefläche erstreckt und welches eine homogene Feldstärke aufweist.

Die Seitenteile sind entlang einer Haupterstreckungsrichtung der Liege in horizontaler Richtung verschiebbar.

Die Erfindung betrifft insbesondere eine Vorrichtung, bei welcher die Seitenteile nur einen Teilbereich der Länge der Liege einnehmen, insbesondere einen Teilbereich, welcher weniger als die Hälfte lang ist, als die Liege selbst.

Das zwischen den Seitenteilen vorhandene Behandlungsvolumen erstreckt sich somit auch nur über einen Teilbereich der Liegefläche und die Seitenteile müssen je nachdem, welche Körperregion behandelt werden soll, verschoben werden.

Gemäß der Erfindung sind die Seitenteile über einen unter der Liegefläche angeordneten Träger gekoppelt, so dass sich diese gleichzeitig verschieben.

Diese Anordnung hat den Vorteil, dass durch Bewegen eines Seitenteils von einer Seite der Vorrichtung her das auf der gegenüberliegenden Seite angeordnete Seitenteil mitbewegt wird, was die Bedienung erleichtert.

Gleichzeitig ist durch den Träger sichergestellt, dass die Seitenteile in einer festen Position gegenüberliegend angeordnet sind, so dass diese nicht in eine Position gebracht werden können, in welcher sich die Seitenteile nicht genau gegenüberstehen, was zur Folge hätte, dass aufgrund der nicht mehr vorhandenen Helmholtz-Konfiguration das gewünschte geradlinige und homogene Feld nicht erzeugt wird.

Gleichzeitig kann, wie es bei einer bevorzugten Ausführungsform vorgesehen ist, die Vorrichtung oben über den Seitenteilen offen sein, d.h. die Seitenteile sind vorzugsweise nur unterhalb, nicht aber oberhalb der Liegefläche verbunden.

Vorzugsweise ist der Träger auf Schienen verschiebbar.

Bei einer bevorzugten Ausführungsform der Erfindung umfasst der Träger Gleitelemente, die eine Ausnehmung aufweisen, in welche die Schienen greifen.

Schienen und/oder Gleitelemente können eine Beschichtung aufweisen, welche die Reibung herabsetzt. Weiter können insbesondere die Gleitelemente aus einem Kunststoff bestehen, der eine niedrige Reibung verursacht.

Durch die Verwendung von Gleitelementen können die Seitenteile zusammen mit dem Träger leicht verschoben werden, wobei gleichzeitig aufgrund der Haftreibung nach dem Loslassen der Träger derart festgesetzt ist, dass sich dieser nicht unbeabsichtigt verschiebt. Auf zusätzliche Mittel zur Festlegung des Trägers gegenüber der Schiene kann somit vorzugsweise verzichtet werden.

Bei einer alternativen Ausführungsform der Erfindung kann der Träger auch Räder oder Rollen umfassen, die jeweils auf einer Schiene laufen.

Bei einer weiteren Ausführungsform der Erfindung ist der Träger verriegelbar. Dies kann z.B. durch in eine Schiene greifende Formschlusselemente realisiert sein. So kann der Träger während einer Behandlung nicht mehr verschoben werden und es ist sichergestellt, dass sich die Position des Behandlungsvolumens nicht ändert.

Bei einer Weiterbildung der Erfindung umfasst die Vorrichtung eine austauschbare Liegefläche. Die Liegefläche kann so gegen eine andere Liegefläche mit einer anderen Form oder mit weiteren funktionellen Behandlungskomponenten, z.B. Gurte zur Fixierung eines Körperteils, getauscht werden.

Bei einer Weiterbildung der Erfindung umfasst die Vorrichtung eine Beleuchtungseinrichtung mit veränderbarer Farbe. Diese ist vorzugsweise als LED-Leuchtstreifen ausgebildet.

Es ist insbesondere vorgesehen, dass sich im Betrieb der Vorrichtung, also beim Erzeugen von Kernspinresonanzen, die Lichtfarbe ändert.

Weiter kann die Beleuchtungseinrichtung der Markierung des Behandlungsvolumens dienen, z.B. indem zwei Leuchtstreifen, die an Seitenteilen angeordnet sind, seitliche Lichtstreifen auf die Liegefläche projizieren.

Gemäß der Erfindung umfasst die Liege ein Gehäuse, welches eine Ausnehmung umfasst, durch welche sich der Träger erstreckt. Der Träger ist so unterhalb der Liegefläche durch das Gehäuse der Liege hindurchgeführt.

IDie Ausnehmung ist als horizontal verlaufender Schlitz ausgebildet, entlang dessen die Seitenteile verschoben werden können.

Das Gehäuse der Liege erstreckt sich horizontal sowohl oberhalb als auch unterhalb der Ausnehmung. Hierdurch wird u.a. das Risiko reduziert, dass bewegliche Teile, insbesondere bewegliche Teile des Trägers, versehentlich gegen die Gliedmaßen eines Benutzers geschoben werden, wenn dieser beispielsweise auf der Liege sitzt.

Die Ausnehmung, insbesondere der horizontal verlaufende Schlitz, ist vorzugsweise von der Oberseite und der Unterseite in etwa gleich weit beabstandet, insbesondere beträgt der Unterschied des Abstands des Schlitzes von der Unterseite gegenüber der Oberseite weniger als 30%.

Die Seitenteile sind bei einer bevorzugten Ausführungsform entlang eines Bereiches der Liege verschiebbar, der eine Länge von zumindest 60%, vorzugsweise von zumindest 75% der Länge der Liegefläche hat.

So lassen sich die Seitenteile und damit das Behandlungsvolumen entlang nahezu der gesamten Länge der Liege verschieben und es kann jede Körperregion eines Benutzers behandelt werden, ohne dass der Benutzer seine Position auf der Liege wechseln muss.

Gemäß der Erfindung ist der Träger als verschiebbarer Rahmen ausgebildet, welcher sich zur Seite hin erstreckende Ausleger umfasst. An den Auslegern sind die Seitenteile angeschlagen.

Weiter umfasst der Träger zumindest einen sich vertikal in Richtung der Liegefläche erstreckenden Abschnitt, an welchem eine in einer horizontalen Ebene flachliegende weitere Spule angebracht ist.

Durch den Gegenstand der Erfindung wird zusammen mit dem Seitenteil und dem Träger gleichzeitig eine weitere Spule unterhalb der Liegefläche bewegt.

Diese weitere Spule ist vorzugsweise dazu ausgebildet, ein magnetisches Wechselfeld in das Behandlungsvolumen einzustrahlen, welches senkrecht zu dem Feld steht, welches von den Spulen in den Seitenteilen erzeugt wird.

Die Vorrichtung ist vorzugsweise derart ausgebildet, dass die Feldstärke des von den Seitenteilen erzeugten Feldes 0,1 bis 100 mT, besonders bevorzugt 0,3 bis 3 mT beträgt. Dementsprechend beträgt die Frequenz des senkrecht hierzu eingestrahlten Wechselfeldes vorzugsweise 1 bis 100 kHz, bevorzugt 10 bis 100 kHz. Die maximale Feldstärke des Wechselfeldes liegt vorzugsweise zwischen 0,1 bis 100 mT, besonders bevorzugt 0,1 und 3 mT.

Bei einer Weiterbildung der Erfindung ist ein Steuergerät zur Ansteuerung der Spulen an dem Träger angebracht oder mit dem Träger verbunden.

Gemäß einer bevorzugten Ausführungsform der Erfindung sind sämtliche elektrischen und elektronischen Komponenten zur Steuerung der Vorrichtung und insbesondere zur Versorgung der Spulen mit Strom mit dem Träger verbunden.

So muss nur eine elektrische Versorgung bereitgestellt werden, welche mit dem Träger verbunden ist.

Eine elektrische Verbindung zwischen der Liege und den beweglichen Teilen in Form sämtlicher Teile, die am Träger angebracht sind, ist dagegen nicht erforderlich.

Bei einer Weiterbildung der Erfindung ist an einem Seitenteil eine Bedieneinheit angebracht. Die Bedieneinheit ist vorzugsweise in einem oberen Bereich eines Seitenteils angebracht und so gut zugänglich.

Die Bedieneinheit kann insbesondere einen Bildschirm, insbesondere ausgebildet als Touchscreen, umfassen. Nachfolgend wird ein Verfahren erläutert, mittels dessen eine Vorrichtung, wie sie vorstehen beschrieben wurde, betrieben werden kann.

Mittels zwei sich gegenüberliegender Spulen wird, wie zuvor dargestellt, ein erstes Magnetfeld erzeugt, wobei mit zumindest einer weiteren Spule ein zweites Magnetfeld erzeugt wird, das innerhalb eines Behandlungsvolumens senkrecht zu dem Magnetfeld eingestrahlt wird, welches durch die sich gegenüberliegenden Spulen erzeugt wird.

Dieses Magnetfeld ist als magnetisches Wechselfeld ausgebildet, dessen Frequenz auf die Feldstärke des anderen Feldes derart abgestimmt ist, dass die Resonanzbedingung für Kernspinresonanzen zumindest zeitweist erzielt wird.

Es wird zumindest in einer Einschaltphase der Vorrichtung die Stärke des durch die sich gegenüberliegenden Spulen fließenden Stroms und/oder die Frequenz des magnetischen Wechselfeldes gemessen.

Die Stärke eines von einer Spule erzeugten magnetischen Feldes ist proportional zum Strom. Durch eine Messung des Stroms kann daher unmittelbar auf die Feldstärke geschlossen werden.

Der Strom wird vorzugsweise durch Spannungsabgriff an einem in Reihe geschalteten, niederohmigen Widerstand bestimmt.

Liegt im Betrieb die Stromstärke nicht in einem vorgegebenen und in der Vorrichtung gespeicherten Sollbereich, der insbesondere einprogrammiert sein kann, so kann auf einen Defekt an den Spulen oder an der Elektronik zur Ansteuerung der Spulen geschlossen werden.

Alternativ oder zusätzlich wird auch die Frequenz des magnetischen Wechselfeldes überwacht. Dies kann beispielsweise digital mittels eines Mikroprozessors erfolgen, über den das elektrische Signal, das an der Spule zur Erzeugung eines Wechselfeldes anliegt, abgetastet wird und mittels dessen überprüft werden kann, ob das Wechselfeld im gewünschten Frequenzbereich liegt.

Durch die Überwachung der Feldstärke der Sweepspulen sowie durch die Überwachung der Frequenz der Spule, die das Wechselfeld erzeugt, können Fehler erfasst werden und es wird sichergestellt, dass die Resonanzbedingung im Behandlungsvolumen immer erreicht wird.

Bei einer bevorzugten Ausführungsform wird die Stärke des durch die sich gegenüberliegenden Spulen fließenden Stroms oder die Frequenz des magnetischen Wechselfeldes während des Betriebs der Vorrichtung wiederkehrend gemessen.

Eine Überprüfung erfolgt mithin nicht nur initial während der Einschaltphase, sondern fortlaufend während des Betriebs, so dass auch erst während des Betriebs auftretende Fehler, welche insbesondere erst durch eine Erwärmung von elektrischen Bauteilen entstehen können, erfasst werden.

Bei einer Weiterbildung wird die Stärke des durch die sich gegenüberliegenden Spulen fließenden Stroms getrennt für jede Spule einzeln gemessen.

Dies ist messtechnisch einfacher und ermöglicht zudem zumindest die Erkennung, auf welcher Seite der Fehler liegt.

Falls die Stärke des durch die sich gegenüberliegenden Spulen fließenden Stroms und/oder die Frequenz des magnetischen Wechselfeldes nicht innerhalb eines gespeicherten Sollbereichs liegt, innerhalb dessen sichergestellt ist, dass die Resonanzbedingung erreicht wird, wird vorzugsweise der Betrieb der Vorrichtung abgebrochen und eine Fehlermeldung generiert.

Die Fehlermeldung kann beispielsweise auf einem Bildschirm ausgegeben werden und der Verwender wird aufgefordert, die Vorrichtung zu überprüfen und neu zu starten.

### Kurzbeschreibung der Zeichnungen

Der Gegenstand der Erfindung soll im Folgenden bezugnehmend auf die Zeichnungen Fig. 1 bis Fig. 8 anhand eines Ausführungsbeispiels näher erläutert werden.
Fig. 1 und Fig. 2 sind perspektivische Ansichten eines Ausführungsbeispiels einer erfindungsgemäßen Vorrichtung zur Kernspinresonanztherapie.
Fig. 3 ist eine perspektivische Detailansicht.
Fig. 4 zeigt die Vorrichtung, wobei das Gehäuse der Liege abgenommen ist.
Fig. 5 ist eine Detailansicht auf den Träger der Vorrichtung.
Fig. 6 ist eine perspektivische Ansicht, wobei sowohl das Gehäuse der Liege als auch die Gehäuseteile der Seitenteile ausgeblendet sind.
Fig. 7 ist eine aufgeschnittene Detailansicht der Vorrichtung entlang des Trägers.
Fig. 8 ist ein Flussdiagramm, in welchem ein Verfahren zum Betrieb einer Vorrichtung erläutert wird.

### Detaillierte Beschreibung der Zeichnungen

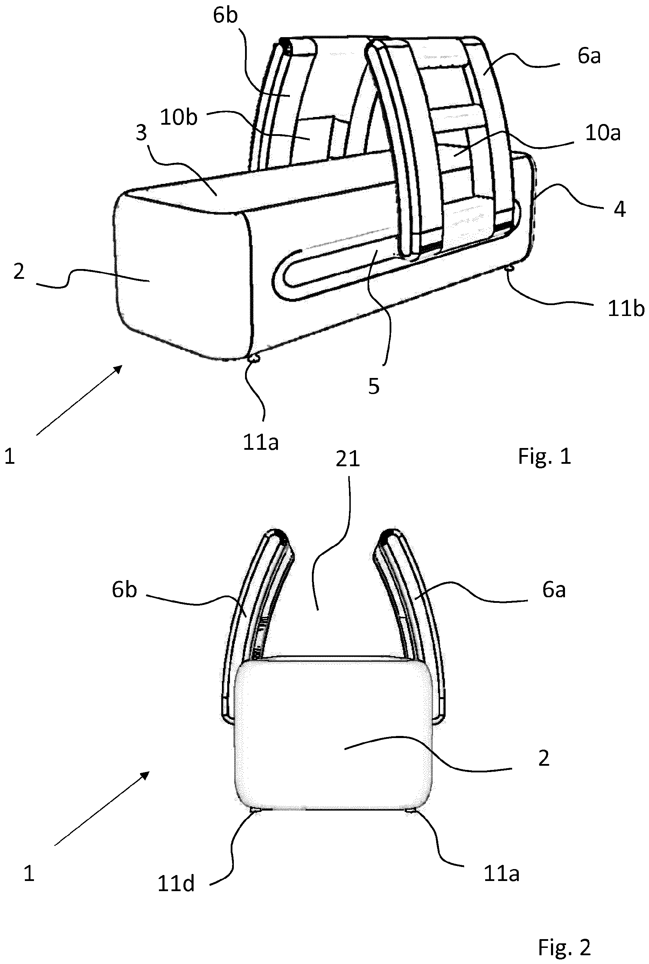

Fig. 1 zeigt eine perspektivische Ansicht einer Vorrichtung 1 zur Erzeugung von Kernspinresonanzen im Gewebe eines Benutzers.

Die Vorrichtung 1 umfasst eine Liege 2 mit einem in diesem Ausführungsbeispiel im Wesentlichen quaderförmig ausgestalteten Gehäuse 4.

Auf der Liege 2 befindet sich die horizontal ausgerichtete Liegefläche 3.

Das Gehäuse der Liege 2 umfasst eine in diesem Ausführungsbeispiel schlitzförmige Ausnehmung 5, neben welcher die sich vertikal nach oben erstreckenden Seitenteile 6a und 6b herausragen.

Die Seitenteile 6a und 6b sind so neben der Liegenfläche 3 angeordnet.

Die Seitenteile 6a und 6b können in Haupterstreckungsrichtung der Liege entlang der Ausnehmung 5 hin- und hergeschoben werden. Hierzu erstreckt sich die Ausnehmung vorzugsweise über mindestens 70% der Länge der Liege 2.

In den Ausnehmungen 6a und 6b ist jeweils eine Spule (hier nicht dargestellt) angeordnet. So kann ein magnetisches Feld in dem zwischen den Seitenteilen 6a und 6b liegenden Behandlungsvolumen erzeugt werden, welches sich geradlinig von einem Seitenteil 6a zum anderen Seitenteil 6b erstreckt und welches bezogen auf die Feldstärke eine hohe Homogenität hat.

Die Seitenteile 6a und 6b umfassen eine zentrale Öffnung, 10a, 10b.

Weiter zu sehen sind die Füße 11a und 11b, auf denen die Liege 2 ruht.

Fig. 2 ist eine Ansicht auf die Stirnseite der Vorrichtung 1 zur Kernspinresonanztherapie.

Zu erkennen ist, dass die sich vertikal nach oben erstreckenden Seitenteile 6a und 6b nach oben hin aufeinander zulaufen.

Die Seitenteile 6a und 6b sind des Weiteren gekrümmt ausgebildet.

Das zwischen den Seitenteilen 6a und 6b vorhandene Behandlungsvolumen 21 ist so optimal an die Anatomie eines Benutzers angepasst.

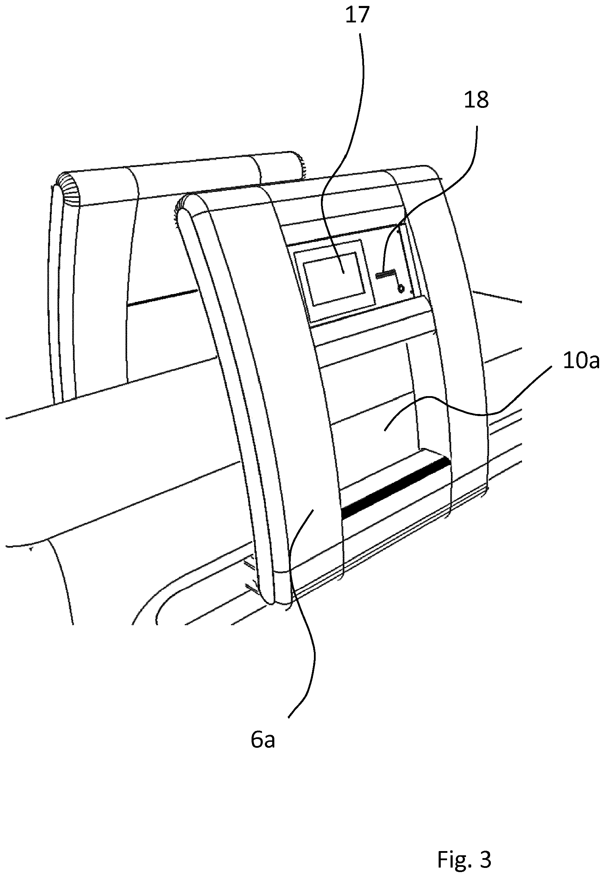

Fig. 3 ist eine perspektivische Detailansicht der Vorrichtung zur Kernspinresonanztherapie, in welcher insbesondere das Seitenteil 6a gut zu erkennen ist.

Zu erkennen ist, dass das Seitenteil 6a im oberen Bereich einen Bildschirm 17 aufweist, welcher vorzugsweise als Touchscreen ausgebildet ist und welcher der Bedienung der Vorrichtung dient.

Der Bildschirm 17 ist in diesem Ausführungsbeispiel über der Öffnung 10a angeordnet.

Neben dem Bildschirm 17 befindet sich in diesem Ausführungsbeispiel ein Kartenleser 18. Über Speicherkarten kann eine Benutzeridentifikation erfolgen. Weiter können auf einer Speicherkarte Programme zum Betrieb der Vorrichtung abgelegt sein.

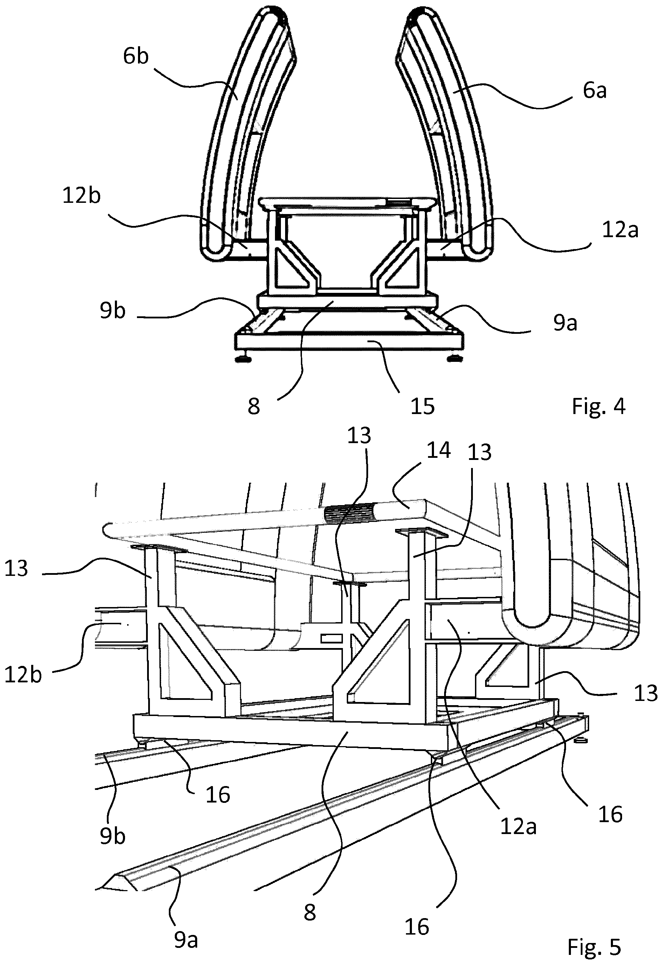

Fig. 4 ist eine Draufsicht auf die Stirnseite der Vorrichtung, bei welcher das Gehäuse der Liege (4 in Fig. 1) ausgeblendet ist.

Zu erkennen ist, dass im Gehäuse der Liege ein Träger 8 angerordnet ist, welcher auf Schienen 9a, 9b linear verschiebbar ist. Die Schienen 9a und 9b sind Teil eines Rahmens 15 des Fußteils.

Seitlich vom Träger 8 erstrecken sich in horizontaler Richtung die Ausleger 12a und 12b, an denen die Seitenteile 6a und 6b angebracht sind.

So ist das Seitenteil 6a über den Träger 8 mit dem Seitenteil 6b gekoppelt und durch Ziehen an einem Seitenteil werden beide Seitenteile verschoben.

Fig. 5 ist eine perspektivische Detailansicht auf den Träger 8.

Zu erkennen ist, dass der Träger 8 an seiner Unterseite an seinen Ecken Gleitelemente 16 umfasst, welche eine Ausnehmung aufweisen, in die die Schienen 9a und 9b jeweils greifen.

Die Schienen 9a und 9b sowie Gleitelemente 16 bestehen vorzugsweise aus einem Material oder sind mit einer Beschichtung aus einem Material versehen, welches für eine niedrige Gleitreibung sorgt.

Zu erkennen ist weiter, dass der Träger 8 sich vertikal nach oben erstreckende Abschnitte 13 umfasst, an deren Ende eine weitere Spule 14 angeordnet ist.

Die Spule 14 liegt flach unter der Liegefläche und wird zusammen mit dem Träger 8 und den Seitenteilen bewegt.

Über die Spule 14 wird ein magnetisches Wechselfeld in die Behandlungszone (21 in Fig. 2) eingestrahlt, welches senkrecht zu dem Magnetfeld steht, welches von den Spulen in den Seitenteilen erzeugt wird.

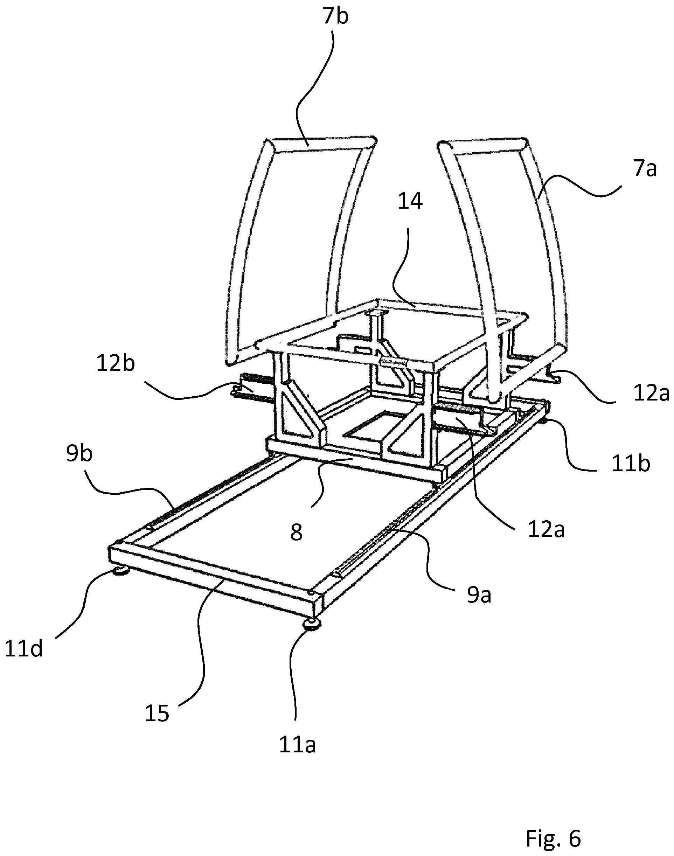

Fig. 6 zeigt eine perspektivische Ansicht, in welcher sämtliche Gehäusekomponenten, also auch die Gehäusebestandteile der Seitenteile, ausgeblendet sind.

Zu erkennen ist, dass die Vorrichtung im Inneren auf einem Rahmen 15 ruht, welcher auf Füßen 11a bis 11d steht und auf welchem die Schienen 9a und 9b entlang der Haupterstreckungsrichtung der Vorrichtung verlaufen.

Auf dem Rahmen 15 ist der Träger 8 verschiebbar angeordnet.

Der Träger 8 ist über die Ausleger 12a sowie 12b sowie weitere hier ausgeblendete Gehäusekomponenten mit den Spulen 7a und 7b verbunden, welche in den Seitenteilen der Vorrichtung angeordnet sind.

Die Spulen 7a und 7b erstrecken sich bis unter die Oberseite der Liegefläche (3 in Fig. 1).

Die Spule 14 zur Erzeugung eines magnetischen Wechselfeldes liegt höher als das untere Ende der Spulen 7a und 7b, aber ist gleichzeitig unterhalb der vertikalen Mitte der Spulen 7a und 7b angeordnet.

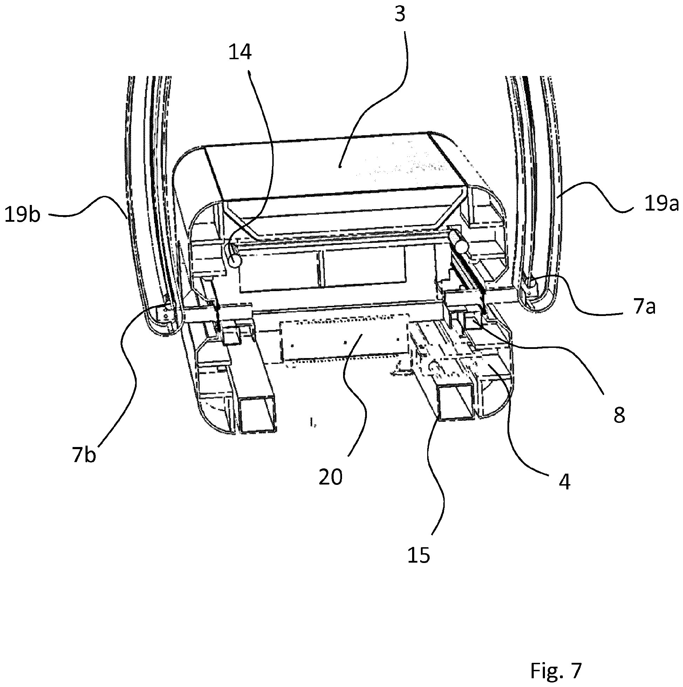

Fig. 7 ist eine aufgeschnittene perspektivische Detailansicht, in welcher insbesondere der Träger 8 zu sehen ist.

Zu sehen ist in dieser Detailansicht, dass die Spulen 7a und 7b jeweils von einem Gehäuse 19a, 19b umgeben sind.

Das Gehäuse 4 der Liege erstreckt sich in vertikaler Richtung bis zum Rahmen 15 sowie bis zur Liegefläche 3.

Zu erkennen ist dieser Detailansicht auch, dass der verschiebbare Rahmen 8 ein Steuergerät 20 umfasst, welches sämtliche elektronischen und elektrischen Komponenten zur Ansteuerung der Spulen (7a und 7b sowie 14) umfasst.
Das Steuergerät 20 ist über elektrische Leitungen (nicht dargestellt) mit dem in Fig. 3 gezeigten Bildschirm verbunden, über welche die Vorrichtung bedient wird.

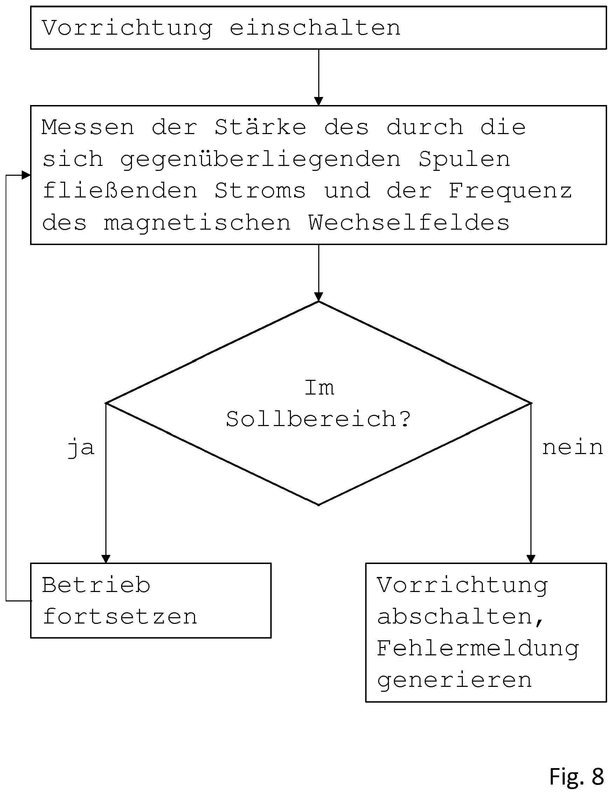

Fig. 8 ist ein Flussdiagramm, anhand dessen das Verfahren zum Betrieb einer Vorrichtung zur Kernspinresonanztherapie näher erläutert werden soll. Die Vorrichtung wird derart gesteuert, dass nach dem Einschalten die Stärke des Stroms gemessen wird, welcher durch die sich gegenüberliegenden Spulen fließt. Insbesondere wird der Strom der in den Seitenteilen der zuvor beschriebenen Vorrichtungen vorhandenen Spulen gemessen.
Weiter wird am Anschluss der Spule zur Einstrahlung des magnetischen Wechselfeldes die Frequenz, beispielsweise über einen integrierten Schaltkreis, gemessen.
In der Vorrichtung sind Sollbereiche bzw. Schwellwerte sowohl für die Stromstärke als auch für die Frequenz abgespeichert.

Liegt Stromstärke oder Frequenz einer Spule bereits beim Einschalten außerhalb des Sollbereiches, wird die Vorrichtung abgeschaltet und eine Fehlermeldung generiert.

Falls sowohl Frequenz als auch Stromstärke im Sollbereich liegen, kann der Betrieb fortgesetzt werden, wobei der Verfahrensschritt des Messens der Stromstärke und der Frequenz auch während des Betriebs der Vorrichtung wiederkehrend wiederholt wird. Diese Messschleife wird nur dann unterbrochen, wenn bei einer Messung Frequenz oder Stromstärke nicht im Sollbereich liegen, wobei sodann die Vorrichtung wiederum abgeschaltet und eine Fehlermeldung generiert wird.
Durch die Erfindung konnte eine Vorrichtung zur Kernspinresonanztherapie auf einfache Weise leichter und sicherer bedienbar ausgestaltet werden.

### Bezugszeichenliste

- 1: Vorrichtung
- 2: Liege
- 3: Liegefläche
- 4: Gehäuse
- 5: Ausnehmung
- 6a, 6b: Seitenteile
- 7a, 7b: Spule
- 8: Träger
- 9a, 9b: Schiene
- 10a, 10b: Öffnung
- 11a-11d: Fuß
- 12a, 12b: Ausleger
- 13: Abschnitt
- 14: Spule
- 15: Rahmen
- 16: Gleitelement
- 17: Bildschirm
- 18: Kartenleser
- 19a, 19b: Gehäuse
- 20: Steuergerät
- 21: Behandlungsvolumen

## Patentansprüche

1. Vorrichtung (1) zur Kernspinresonanztherapie, umfassend eine Liege (2) mit einer Liegefläche (3) für den Benutzer, wobei die Liege (2) seitlich angrenzend an die Liegefläche (3) zwei gegenüberliegende Seitenteile (6a, 6b) aufweist, welche jeweils eine Spule (7a, 7b) umfassen und welche entlang einer Haupterstreckungsrichtung der Liege (2) in horizontaler Richtung verschiebbar sind,
wobei die Seitenteile (6a, 6b) über einen unter der Liegefläche (3) angeordneten Träger (8) gekoppelt sind, so dass sich diese gleichzeitig verschieben, **dadurch gekennzeichnet, dass** die Liege (2) ein Gehäuse (4) aufweist, welches eine Ausnehmung (5) umfasst, durch welche sich der Träger erstreckt, wobei die Ausnehmung (5) als horizontal verlaufender Schlitz ausgebildet ist, und wobei der Träger (8) als verschiebbarer Rahmen ausgebildet ist, welcher sich zur Seite erstreckende Ausleger (12a, 12b) umfasst, an welchen die Seitenteile (6a, 6b) angeschlagen sind, wobei der Träger (8) zumindest einen sich vertikal in Richtung der Liegefläche (3) erstreckenden Abschnitt (13) umfasst, an welchem eine in einer horizontalen Ebene flach liegende weitere Spule (14) angebracht ist.

2. Vorrichtung (1) zur Kernspinresonanztherapie nach dem vorstehenden Anspruch, **dadurch gekennzeichnet, dass** der Träger (8) auf Schienen (9a, 9b) verschiebbar ist.

3. Vorrichtung (1) zur Kernspinresonanztherapie nach einem der vorstehenden Ansprüche, **dadurch gekennzeichnet, dass** die Seitenteile (6a, 6b) entlang eines Bereichs der Liege (2) verschiebbar sind, der eine Länge von zumindest 60 %, vorzugsweise von zumindest 75 % der Länge der Liegefläche (3) hat.

4. Vorrichtung (1) zur Kernspinresonanztherapie nach einem der vorstehenden Ansprüche, **dadurch gekennzeichnet, dass** ein Steuergerät (20) zur Ansteuerung der Spulen (7a, 7b, 14) an dem Träger (8) angebracht oder mit dem Träger (8) verbunden ist.

5. Vorrichtung (1) zur Kernspinresonanztherapie nach einem der vorstehenden Ansprüche, **dadurch gekennzeichnet, dass** an einem Seitenteil (6a) eine Bedieneinheit anbracht ist, insbesondere eine Bedieneinheit, die einen Bildschirm (17) umfasst.

## Claims

1. A device (1) for nuclear magnetic resonance therapy, comprising
a bed (2) with a bed surface (3) for the user, wherein the bed (2) has two opposite side parts (6a, 6b) laterally adjoining the bed surface (3), each side part including a coil (7a, 7b) and being displaceable in a horizontal direction along a main extension direction of the bed (2); wherein the side parts (6a, 6b) are coupled to one another through a mounting structure (8) arranged below the bed surface (3) such that they move simultaneously;
**characterized in that** the bed (2) comprises a housing (4) which has a recess (5) through which the mounting structure extends, wherein said recess (5) is in the form of a horizontally extending slot, and wherein the mounting structure (8) is in the form of a slideable frame which comprises sideways extending beams (12a, 12b) to which the side parts (6a, 6b) are mounted; wherein the mounting structure (8) comprises at least one portion (13) extending vertically toward the bed surface (3), on which a further coil (14) is mounted so as to lie flat in a horizontal plane.

2. The device (1) for nuclear magnetic resonance therapy according to the preceding claim, wherein the mounting structure (8) is displaceable on rails (9a, 9b).

3. The device (1) for nuclear magnetic resonance therapy according to any one of the preceding claims, wherein the side parts (6a, 6b) are displaceable along a portion of the bed (2), said portion having a length corresponding to at least 60 %, preferably at least 75 % of the length of the bed surface (3).

4. The device (1) for nuclear magnetic resonance therapy according to any one of the preceding claims, wherein a control device (20) for controlling the coils (7a, 7b, 14) is mounted to the mounting structure (8) or connected to the mounting structure (8).

5. The device (1) for nuclear magnetic resonance therapy according to any one of the preceding claims, wherein an operating unit is mounted to one side part (6a), in particular an operating unit comprising a screen (17).

## Revendications

1. Dispositif (1) de thérapie par résonance magnétique nucléaire, comprenant une table (2) dotée d'un plan de couchage (3) pour l'utilisateur, la table (2) présentant, sur les côtés et de façon contiguë au plan de couchage (3), deux éléments latéraux (6a, 6b) qui se font face et comportent chacun une bobine (7a, 7b) et qui peuvent être déplacés dans le sens horizontal, le long d'une dimension principale de la table (2), les éléments latéraux (6a, 6b) étant accouplés par l'intermédiaire d'un support (8) placé sous le plan de couchage (3), de sorte qu'ils se déplacent en même temps, **caractérisé en ce que** la table (2) comprend une enveloppe (4) qui présente un évidement (5) à travers lequel s'étend le support, ledit évidement (5) étant réalisé sous la forme d'une fente horizontale, et ledit support (8) étant réalisé sous la forme d'un cadre mobile doté de bras (12a, 12b) qui s'étendent vers le côté et sur lesquels sont montés les éléments latéraux (6a, 6b), le support (8) comprenant au moins une partie (13) qui s'étend verticalement en direction du plan de couchage (3) et sur laquelle est fixée une bobine (14) supplémentaire disposée à plat dans un plan horizontal.

2. Dispositif (1) de thérapie par résonance magnétique nucléaire selon la revendication précédente, dans lequel le support (8) peut être déplacé sur des rails (9a, 9b).

3. Dispositif (1) de thérapie par résonance magnétique nucléaire selon l'une des revendications précédentes, dans lequel les éléments latéraux (6a, 6b) peuvent être déplacés le long d'une partie de la table (2) qui présente une longueur correspondant au moins à 60 %, de préférence au moins à 75 %, de la longueur du plan de couchage (3).

4. Dispositif (1) de thérapie par résonance magnétique nucléaire selon l'une des revendications précédentes, dans lequel un appareil de contrôle (20), destiné à activer les bobines (7a, 7b, 14), est fixé au support (8) ou est relié au support (8).

5. Dispositif (1) de thérapie par résonance magnétique nucléaire selon l'une des revendications précédentes, dans lequel une unité de commande est prévue sur un élément latéral (6a), en particulier une unité de commande qui comprend un écran (17).
